Europäisches Patentamt

European Patent Office

Office européen des brevets

(19)

(11) Publication number: **0 339 568**
**A1**

# (12) EUROPEAN PATENT APPLICATION

(21) Application number: **89107458.5**

(22) Date of filing: **25.04.89**

(51) Int. Cl.⁴: **C12N 15/00 , C12P 21/00 , C12N 1/16 , //(C12N1/16, C12R1:84)**

The applicant has filed a statement in accordance with Rule 28 (4) EPC (issue of a sample only to an expert). Accession numbers of the deposits: NRRL Y-15851, NRRL B-18114, NRRL B-15867, NRRL B-15868, NRRL B-15869, NRRL B-15890 and NRRL B-18016.

The microorganisms have been deposited with The Northern Regional Research Center under numbers NRRL Y-15851, B-18114, B-15867, B-15868, B-15869, B-15890 and B-18016.

(30) Priority: **25.04.88 US 186410**

(43) Date of publication of application:
**02.11.89 Bulletin 89/44**

(84) Designated Contracting States:
**AT BE CH DE ES FR GB GR IT LI LU NL SE**

(71) Applicant: **PHILLIPS PETROLEUM COMPANY**
**5th and Keeler**
**Bartlesville Oklahoma 74004(US)**

(72) Inventor: **McCombie, William Richard**
**1516 Kings Drive 1217**
**Bartlesville, OK 74006(US)**
Inventor: **Davis, Frances Marie**
**10301 Stella Link**
**Houston, TX 77025(US)**
Inventor: **Hubbard, Cynthia Elise**
**Rt. 1 Box 768**
**Shepherdstown, WV 25443(US)**
Inventor: **Divelbiss, Deborah Kay**
**621 SE 13th Apt.N**
**Bartlesville, OK 74003(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) Expression of Human Interleukin-2 in Methylotrophic Yeasts.

(57) A process for the enhanced production of interleukin-2. Also disclosed are novel DNA molecules and methylotrophic yeasts transformed with these molecules.

FIG. 2

EP 0 339 568 A1

## Expression of Human Interleukin-2 in Methylotrophic Yeasts

### Field of Invention

This invention relates to the field of recombinant DNA biotechnology. In one aspect, this invention relates to a process for the expression of human interleukin-2 (hIL-2) protein in methylotrophic yeasts. In another aspect the present invention relates to novel DNA molecules and novel yeast strains transformed therewith.

### Background

Human interleukin-2 (hIL-2) is a protein produced by T-cells that have been activated by mitogens or antigens. hIL-2 mediates many immune responses and promotes long-term proliferation of T-cells and B-cells following interaction with an antigen. These characteristics of hIL-2 have sparked interest in using this protein in the treatment of immunodeficient patients. Therefore, hIL-2 has become an important protein for biotechnological research and production.

Unfortunately although hIL-2 has been produced in E. coli, mammalian cells, Saccharomyces cerevisiae, and Streptomyces lividans, each of these systems has significant disadvantages. For example, E. coli produces endotoxins which must be removed by purification; mammalian cell lines are expensive to grow compared to yeasts or bacteria and the current production of hIL-2 in Streptomyces lividans and Saccharomyces cerevisiae is unacceptably low.

Thus it would be a significant contribution to the art to develop an enhanced process for the production of hIL-2.

Therefore, it is an object of this invention to provide a process for the enhanced production of hIL-2.

Yet another object of this invention is to provide novel vectors containing DNA sequences which code for the hIL-2.

A further object of this invention is to provide novel methylotrophic yeasts transformed with a vector or vectors capable of enhanced production of the hIL-2.

These and other objects of the invention will become apparent from the disclosure and claims herein.

### Summary of the Invention

In accordance with the present invention, I have discovered a process for the enhanced production of hIL-2 comprising transforming a methylotrophic yeast with at least one vector having a compatible expression cassette containing a structural gene for hIL-2 thereof, and culturing the resultant transformants under conditions suitable to obtain the production of hIL-2.

### Detailed Description of the Figures

Figure 1 provides a representation of plasmid pA0804 which contains a linear integrative site-specific vector in the fragment clockwise from BgIII to BgIII. The structural gene may be inserted in the unique EcoRI site of this plasmid.

Figure 2 provides a diagram of the construction of pHIL333 which contains the hIL-2 gene.

### Detailed Description

The hIL-2 structural gene is well known in the art and was first sequenced by Taniguchi et al., Nature 302:305 (1983). The nucleotide sequence of hIL-2 is provided in Table 1. This sequence does not contain the naturally occurring signal sequence of hIL-2 (the first 20 amino acids). The sequence in Table I has a methionine codon inserted in frame with the mature form of hIL-2 which begins at amino acid twenty-one of the published sequence of Taniguchi et al. The structural gene may be obtained by reisolation or preferably synthesized in vitro by a custom gene manufacturer such as British Biotechnology Ltd. Custom synthesized genes are preferred for their ease of use since restriction sites may be inserted or removed in the synthesis of the gene.

The following scheme illustrates only one of numerous ways of modifying and isolating a DNA sequence which contains the hIL-2 structural gene.

The hIL-2 structural gene which was utilized for the practice of the present invention was contained in pUC9:pL IL-2.

The pUC9:pL:IL-2 plasmid was maintained in E. coli strain JM83. However, any E. coli vector and strain system suitable for the maintenance of heterologous genes can be employed for maintaining the hIL-2 gene once isolated.

The nucleotide sequence containing the hIL-2 gene was first prepared for insertion into the expression vector used in the practice of this invention by adding an EcoRI restriction site 5′ to the ATG start codon. The insertion of this second EcoRI site allowed the hIL-2 gene to be recovered as a EcoRI fragment ready for insertion into the unique EcoRI site of the expression vector used in the practice of this invention.

The EcoRI restriction site was contructed by synthesizing an artificial oligonucleotide designed to provide an EcoRI restriction site. This oligonucleotide consisted of the following nucleotide sequences

$$5' \quad CATGAATTCAAAA- \ 3'$$
$$TTAAGTTTTGTAC$$

This synthetic sequence may be produced by either enzymatic or chemical means. Suitable means include but are not limited to chemical procedures based on phosphotriester, phosphite, or phosphoramidite chemistry. The oligonucleotide used in the practice of this invention was synthesized utilizing Applied Biosystems Model 380A DNA Synthesizer following the manufacturer's recommended operating procedures.

The double stranded oligonucleotide was synthesized as single strands of DNA, phosphorylated and annealed to form double stranded DNA as shown above. Plasmid pUC9:pL:hIL-2 was then isolated by standard techniques and digested with NcoI. The NcoI fragment was then dephosphorylated with a suitable reagent such as calf intestine alkaline phosphatase. The linker was then mixed with NcoI fragments and ligated to the NcoI ends with T4 ligase. Successful ligation resulted in regeneration of the plasmid with the linker inserted. The reformed plasmids were selected by utilizing the reaction mixture to transform suitable competent host cells such as DG75′. Transformed cells were selected for by ampicillin resistance. Transformed cells were tested for the presence of the EcoRI fragment by recovering the plasmid DNA from ampicillin resistant colonies followed by EcoRI digestion and gel electrophoresis. Colonies containing the hIL-2 structural gene in an EcoRI fragment were maintained as a source of the gene to be inserted in the expression vector used in the practice of the present invention.

The nucleotide sequence used for the practice of this invention is shown in Table 1.

## Table 1

```
                3         9        15        21        27        33        39        45
                |         |         |         |         |         |         |         |
        1 TAC CAC CAC TAC TAC GTT TTA ACT GAA ACA AAC TGG AGA CTG CCA
          ATG GTG GTG ATG ATG CAA AAT TGA CTT TGT TTG ACC TCT GAC GGT

       46 TGG CAC CTA CTT CAA GTT CTA CAA AGA AAA CAC AGC TAC AAC TGG
          ACC GTG GAT GAA GTT CAA GAT GTT TCT TTT GTG TCG ATG TTG ACC

       91 AGC ATT TAC TGC TGG ATT TAC AGA TGA TTT TGA ATG GAA TTA ATA
          TCG TAA ATG ACG ACC TAA ATG TCT ACT AAA ACT TAC CTT AAT TAT

      136 ATT ACA AGA ATC CCA AAC TCA CCA GGA TGC TCA CAT TTA AGT TTT
          TAA TGT TCT TAG GGT TTG AGT GGT CCT ACG AGT GTA AAT TCA AAA

      181 ACA TGC CCA AGA AGG CCA CAG AAC TGA AAC ATC TTC AGT GTC TAG
          TGT ACG GGT TCT TCC GGT GTC TTG ACT TTG TAG AAG TCA CAG ATC

      226 AAG AAG AAC TCA AAC CTC TGG AGG AAG TGC TAA ATT TAG CTC AAA
          TTC TTC TTG AGT TTG GAG ACC TCC TTC ACG ATT TAA ATC GAG TTT

      271 GCA AAA ACT TTC ACT TAA GAC CCA GGG ACT TAA TCA GCA ATA TCA
          CGT TTT TGA AAG TGA ATT CTG GGT CCC TGA ATT AGT CGT TAT AGT

      316 ACG TAA TAG TTC TGG AAC TAA AGG GAT CTG AAA CAA CAT TCA TGT
          TGC ATT ATC AAG ACC TTG ATT TCC CTA GAC TTT GTT GTA AGT ACA

      361 GTG AAT ATG CTG ATG AGA CAG CAA CCA TTG TAG AAT TTC TGA ACA
          CAC TTA TAC GAC TAC TCT GTC GTT GGT AAC ATC TTA AAG ACT TGT

      406 GAT GGA TTA CCT TTT GTC AAA GCA TCA TCT CAA CAC TAA CTT GAT
          CTA CCT AAT GGA AAA CAG TTT CGT AGT AGA GTT GTG ATT GAA CTA

      451 AAT TAA GTG CTT CCC ACT TAA AAC ATA TCA GGG GGA TCC C
          TTA ATT CAC GAA GGG TGA ATT TTG TAT AGT CCC CCT AGG G
```

Culturing the E. coli strain listed above may be accomplished by any suitable means. General techniques for culturing E. coli are already known in the art and any adaptation of these methods to the specific requirements of the strains used herein is well within the abilities of those skilled in the art.

Recovery of plasmid DNA from E. coli can be accomplished by several techniques due to its compact size and closed spherical superhelical form. For example, following the harvest host cells may be pelleted by centrifugation and then resuspended and lysed. The lysate should be centrifuged to remove cell debris and the supernatant containing DNA retained. A phenol extraction can then be performed to remove most other contaminants from the DNA. The phenol-extracted DNA may then be further treated using a density gradient centrifugation or a gel filtration technique to separate the plasmid DNA from the bacterial DNA. The techniques for achieving the separation alluded to above are well known in the art and numerous methods of performing these techniques are known.

Nuclease digestion of the plasmids may be accomplished by choosing appropriate endonucleases which will cut the selected plasmid in such a way as to facilitate the recovery of the hIL-2 structural gene. The endonucleases used will depend on the plasmid from which the hIL-2 gene is to be excised. For example, the hIL-2 structural gene contained in plasmid pUC9:pL:hIL-2 could be recovered in a NcoI-EcoRI fragment.

Gel electrophoresis of DNA may be accomplished using numerous techniques. See P. G. Sealy and E. M. Southern, Gel Electrophoresis of Nucleic Acids - A Practical Approach (D. Rickwood and B. D. Hames, eds.) p. 39 (1982). Elution may also be accomplished using numerous techniques appropriate for the gel involved, such as electroelution, diffusion, gel dissolution (agarose gels) or physical extrusion (agarose gels). It is additionally recognized that elution may not be necessary with some gels such as high-quality, low melting temperature agarose.

Once the fragment containing the hIL-2 structural gene or fragments thereof is isolated, additional manipulations may be required before it is inserted in the vector. These manipulations may include, but are not limited to the addition of linkers or blunt-ending the fragment.

Following the isolation of the hIL-2 structural gene, the gene is inserted into a suitable methylotrophic yeast vector such as a plasmid. Preferable vectors for the practice of this invention are those compatible with the Pichia genus and most preferably Pichia pastoris.

Plasmids have long been one of the basic elements employed in recombinant DNA technology. Plasmids are circular extrachromosomal double-stranded DNA found in microorganisms. Plasmids have been found to occur in single or multiple copies per cell. Included in plasmid DNA is the information required for plasmid reproduction, i.e. an origin of replication is included for bacterial replication. One or more means of phenotypically selecting the plasmid in transformed cells may also be included in the information encoded in the plasmid. Phenotypic or selection markers, such as antibiotic resistance genes or genes which complement defects in the host biochemical pathways, permit clones of the host cells which have been transformed to be recognized, selected, and maintained.

To express the hIL-2 structural gene in methylotrophic yeasts, the gene must be operably linked to a 5' regulatory region and 3' termination sequence, which forms the expression cassette which will be inserted into the host via a vector.

The following terms are defined herein for the purpose of clarification.

Operably linked--refers to a juxtaposition wherein the components are configured so as to perform their function.

Regulatory region--DNA sequences which respond to various stimuli and affect the rate of mRNA transcription.

3' Termination sequence--sequences 3' to the stop codon which function to stabilize the mRNA such as sequences which elicit polyadenylation. "Pichia compatible" refers to DNA sequences which will perform their normal function in Pichia such as regulatory regions and 3' termination sequences derived from Pichia.

Preferred for the practice of the present invention are integrative vectors, such as the linear site-specific integrative vector of Cregg, as described in European Application Serial Number 86114700.7. Such vectors comprise a serially arranged sequence of at least 1) a first insertable DNA fragment; 2) a selectable marker gene; and 3) a second insertable DNA fragment.

The first and second insertable DNA fragments are each at least about 200 nucleotides in length and have nucleotide sequences which are homologous to portions of the genomic DNA of the species to be transformed. The various components of the integrative vector are serially arranged forming a linear fragment of DNA such that the expression cassette and the selectable marker gene are positioned between the 3' end of the first insertable DNA fragment and the 5' end of the second insertable DNA fragment. The first and second insertable DNA fragments are oriented with respect to one another in the serially arranged linear fragment as they are so oriented in the parent genome.

Nucleotide sequence useful as the first and second insertable DNA fragments are nucleotide sequences which are homologous with separate portions of the native genomic site at which genomic modification is to occur. Thus, for example, if genomic modification is to occur at the locus of the alcohol oxidase gene, the first and second insertable DNA fragments employed must be sequences homologous to separate portions of the alcohol oxidase gene locus. For genomic modification in accordance with the present invention to occur, the two insertable DNA fragments must be oriented with respect to one another in the linear fragment in the same relative orientation as they exist in the parent genome. Examples of nucleotide sequences which could be used as first and second insertable DNA fragments are nucleotide sequences selected from the group consisting of the alcohol oxidase (AOX1) gene, dihydroxyacetone synthase (DHAS) gene, p40 gene and HIS4 gene. The AOX1 gene DHAS gene, p40 gene and HIS4 gene are contained in EP-A- 0183 071.

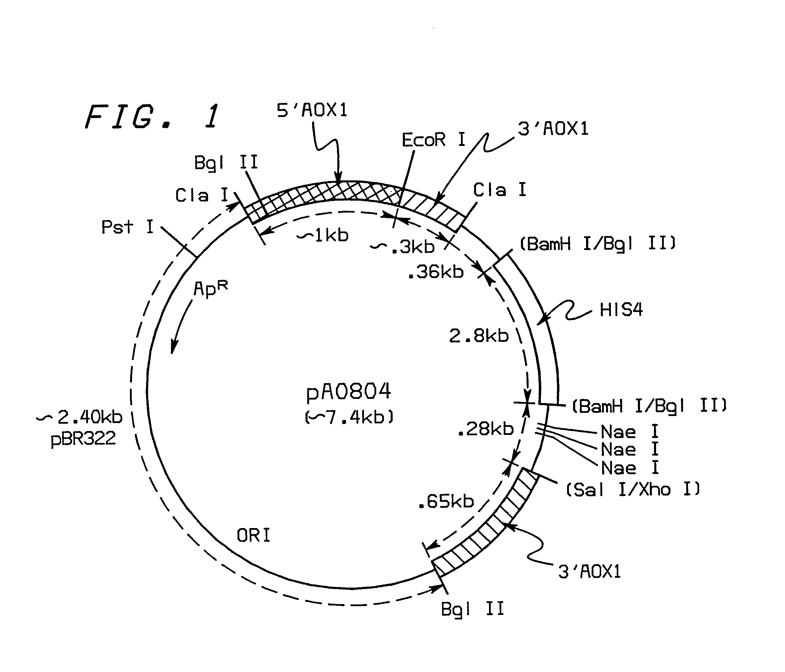

The first insertable DNA fragment may contain an operable regulatory region which may comprise the regulatory region utilized in the expression cassette. The use of the first insertable DNA fragment as the regulatory region for an expression cassette is a preferred embodiment of this invention. Figure 1 provides a diagram of a vector utilizing the first insertable DNA fragment as a regulatory region for a cassette.

Optionally as shown in Figure 1 an insertion site or sites and a 3' termination sequence may be placed immediately 3' to the first insertable DNA fragment. This conformation of the linear site-specific integrative vector has the additional advantage of providing a ready site for insertion of a structural gene without

5

necessitating the addition of a compatible 3' termination sequence.

Additionally the expression vectors used in the practice of this invention could contain a polylinker site to facilitate the insertion of structural genes or cassettes or the like, between the first insertable DNA fragment and the second insertable DNA fragment.

It is also necessary to include at least one selectable marker gene in the DNA used to transform the host strain. This facilitates selection and isolation of those organisms which have incorporated the transforming DNA. The marker gene confers a phenotypic trait to the transformed organism which the host did not have, e.g., restoration of the ability to produce a specific amino acid where the untransformed host strain has a defect in the specific amino acid biosynthetic pathway or resistance to antibiotics and the like.

Exemplary selectable marker genes may be selected from the group consisting of the HIS4 gene and the ARG4 gene from Pichia pastoris and Saccharomyces cerevisiae, the invertase gene (SUC2) from Saccharomyces cerevisiae, the G418R Kanamycin resistance gene from the E. coli transposable elements Tn601 or Tn903.

Those of skill in the art recognize that additional DNA sequences can also be incorporated into the vectors employed in the practice of the present invention, such as for example, bacterial plasmid DNA, bacteriophage DNA, and the like. Such sequences enable the amplification and maintenance of these vectors in bacterial hosts.

If the first insertable DNA fragment does not contain a regulatory region, a suitable regulatory region will need to be inserted operably linked to the structural gene, in order to provide an operable expression cassette. Similarly if no 3' termination sequence is provided at the insertion site to complete the expression cassette, a 3' termination sequence will have to be operably linked to the structural gene to be inserted.

Those skilled in the art are aware of numerous regulatory regions which have been characterized and could be employed in conjunction with methylotrophic yeasts. Exemplary regulatory regions include but are not limited to yeast regulatory regions selected from the group consisting of acid phosphatase, galactokinase, alcohol dehydrogenase, cytochrome c, alpha-mating factor and glyceraldehyde 3-phosphate dehydrogenase regulatory regions isolated from Saccharomyces cerevisiae; the primary alcohol oxidase (AOX1), dihydroxyacetone synthase (DHAS), the p40 regulatory regions, and the HIS4 regulatory region derived from Pichia pastoris and the like. Presently preferred regulatory regions employed in the practice of the present invention are those characterized by their ability to respond to methanol-containing media, such regulatory regions selected from the group consisting of AOX1, DHAS p40 and disclosed in co-pending application serial number 780,102.

The most preferred regulatory region for the practice of this invention is the AOX1 regulatory region.

3' termination sequences may be utilized in the expression cassette or be part of the vector as discussed above. 3' termination sequences may function to terminate, polyadenylate and/or stabilize the messenger RNA coded for by the structural gene when operably linked to a gene. A few examples of illustrative sources for 3' termination sequences for the practice of this invention included but are not limited to the Saccharomyces cerevisiae, Hansenula polymorpha, and Pichia 3' termination sequences. Preferred are those derived from Pichia pastoris such as those selected from the group consisting of the 3' termination sequences of AOX1 gene, DHAS gene, p40 gene and HIS4 gene. And particularly preferred is the 3' termination sequence of the AOX1 gene.

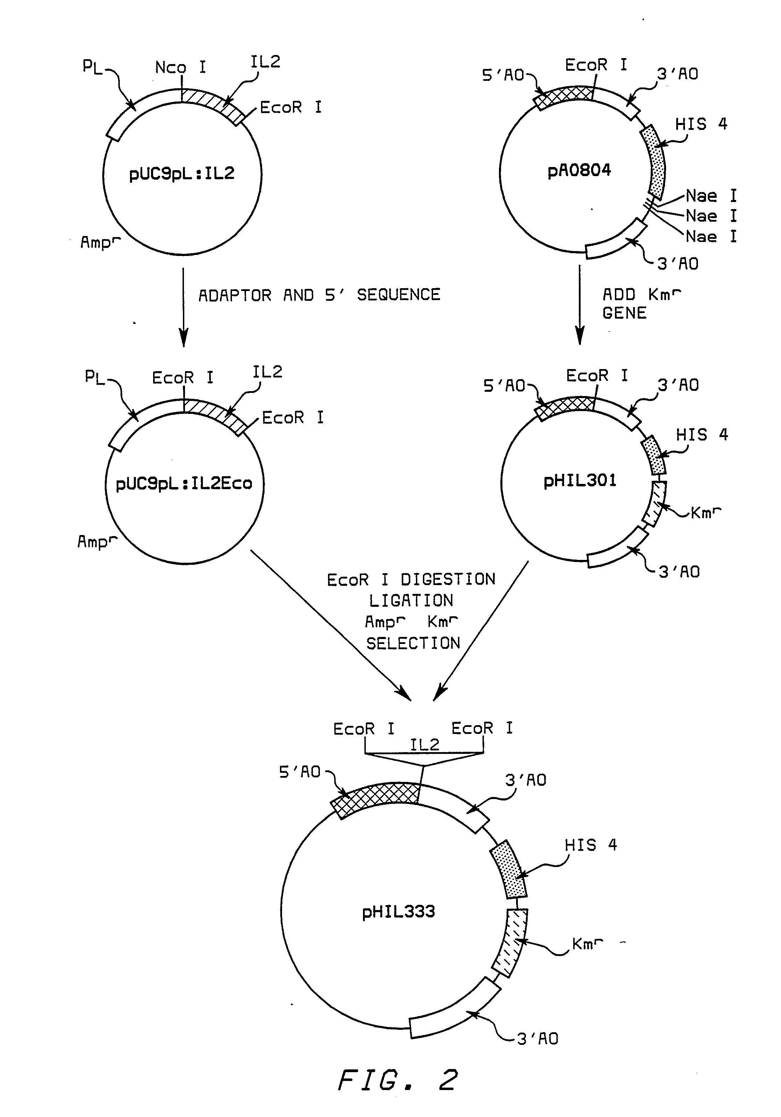

For the practice of the current invention it is currently preferred to use linear site-specific integrative vectors such as the BglII fragments of the constructs shown in Figure 1 and 2. Particularly preferred for the practice of the present invention is the linear vector contained in plasmid pHIL301. Plasmid pHIL301 is a modification of plasmid pAO804 which has had a second selection marker, kanamycin resistance, inserted at Nael site 1 and 3 of pAO804 in the fragment containing the linear site-specific integrative vector. This second selection marker allows for the deletion of the large scale preparation and purification of DNA fragments when isolating the hIL-2 structural gene from pUC9:pL:hIL-2. Instead of digesting pUC9:pL:hIL-2 with EcoRI and the isolating the EcoRI fragment containing the hIL-2 structural gene, this reaction mixture can be immediately ligated into the dephosphorylated EcoRI site of pHIL301. The ligation mixture can then be used to transform competent E. coli cells; plasmids containing pHIL301 can be directly selected for by kanamycin resistance.

The insertion of the hIL-2 structural gene into suitable vectors may be accomplished by any suitable technique which cleaves the vector chosen at an appropriate site or sites and results in at least one · operable expression cassette containing the hIL-2 structural gene being present in the vector.

Ligation of hIL-2 structural gene may be accomplished by any appropriate ligation technique such as utilizing T4 DNA ligase.

The initial selection, propagation, and optional amplification of the ligation mixture of the hIL-2 structural gene and a vector is preferably performed by transforming the mixture into a bacterial host such as E. coli ·

(although the ligation mixture could be transformed directly into a yeast host). Suitable transformation techniques for E. coli are well known in the art. Additionally, selection markers and bacterial origins of replication necessary for the maintenance of a vector in a bacterial host are also well known in the art.

The isolation and/or purification of the desired plasmid containing the hIL-2 structural gene in an expression system may be accomplished by any suitable means for the separation of plasmid DNA from the host DNA.

Similarly the vectors formed by ligation may be tested preferably after propagation to verify the presence of the hIL-2 gene and its operable linkage to a regulatory region and a 3' termination sequence. This may be accomplished by a variety of techniques including but not limited to endonuclease digestion, gel electrophoresis, or endonuclease digestion-Southern hybridization.

Transformation of plasmids or linear vectors into yeast hosts may be accomplished by suitable transformation techniques including but not limited to those taught by Hinnen et al, Proc. Natl. Acad. Sci. 75, (1978) 1929; Ito et al, J. Bacteriol 153, (1983) 163; Cregg et al Mol. Cell Biol. 5 (1985) pg. 3376; or Sreekrishna et al, Gene, 59 (1987) pg. 115. Preferable for the practice of this invention is the transformation technique of Cregg. It is desirable for the practice of this invention to utilize an excess of linear vectors and select for multiple insertions by Southern hybridization.

The yeast host for transformation may be any suitable methylotrophic yeast. Methylotrophic yeast include but are not limited to yeast capable of growth on methanol selected from the genera consisting of Hansenula, Candida, Kloeckera, Pichia, Saccharomyces, Torulopsis and Rhodotorula. A list of specific species which are exemplary of this class of yeasts may be found in C. Anthony, The Biochemistry of Methylotrophs, 269 (1982). Presently preferred are methylotrophic yeasts of the genus Pichia such as the auxotrophic Pichia pastoris GS115 (NRRL Y-15851). Auxotrophic methylotrophic yeasts are also advantageous to the practice of this invention for their ease of selection. It is recognized that wild type methylotrophic yeast strains may be employed with equal success if a suitable transforming marker gene is selected, such as the use of SUC2 to transform Pichia pastoris to a strain capable of growth on sucrose or an antibiotic resistance marker is employed, such as G418.

Transformed methylotrophic yeast cells can be selected for using appropriate techniques including but not limited to culturing previously auxotrophic cells after transformation in the absence of a biochemical product required (due to the cell's auxotrophy), selection by the detection of a new phenotype ("methanol slow"), or culturing in the presence of an antibiotic which is toxic to the yeast in the absence of a resistance gene contained in the transformant.

Isolated transformed methylotrophic yeast cells are cultured by appropriate fermentation techniques such as shake flask fermentation or high density fermentation techniques as disclosed by Cregg, High-Level Expression and Efficient Assembly of Hepatitis B Surface Antigen in the Methylotrophic Yeast, Pichia Pastoris, 5 Bio/Technology 479 (1987).

Expression may be accomplished by methods appropriate to the regulatory region employed. Preferably if methanol responsive regulatory regions are utilized, the induction of expression may be accomplished by exposing the transformed cells in a nutrient media to an appropriate alcohol for the regulatory region employed.

hIL-2 may be recovered in a crude form by lysing transformed cells which have been induced for a sufficient period, using standard techniques such as bead milling, followed by centrifugation sufficient to remove cellular debris. Those of skill in the art are aware of numerous methods available for the extraction of a heterologous protein from unicellular host organisms which could be substituted for the general extraction technique above or for further purification.

The following non-limiting examples are provided to further illustrate the practice of this invention.

## Examples

General information pertinent to the Examples:

### Strains

Pichia pastoris GS115 (HIS4) [NRRL Y-15851] was the host yeast strain used in these examples.

E. coli DG75' (hsd1, leu-6, lacY, thr-1, supE44, tonA21 lambda [-]) or JM107 (endA1, gyrA96, thi1, hsdR 17, SupE44, relA, lambda (-), Δ (lac-ProAB), [F°, traD36, ProAB, lacIqZΔM151] were used for plasmid constructions as well as for propagation of plasmid DNAs.

## Buffers, and Solutions, and Media

The buffers and solutions employed in the following examples have the compositions given below:

dH₂O
deionized H₂O that has been treated with a milli-Q (millipore) reagent water system

1M Tris buffer
121.1 g Tris base in 800 mL of H₂O; adjust pH to the desired value by adding concentrated (35%) aqueous HCl; allow solution to cool to room temperature before final pH adjustment, dilute to a final volume of 1L.

TE buffer
1.0 mM EDTA
in 0.01 M (pH 7.4) Tris buffer

SED
1 M sorbitol
2.5 mM EDTA
50 mM DTT
--adjust to pH 8

SCE
1 M sorbitol
10 mM Sodium citrate
1 mM EDTA
--pH to 5.8 with HCl

CaS
1 M sorbitol
10 mM CaCl₂
10 mM Tris-HCl (pH 7.5)
--filter sterilize

PEG Solution
20% polyethylene glycol-3350
10 mM CaCl₂
10 mM Tris-HCl (pH 7.4)
--filter sterilize

SOS
1M sorbitol
10mM CaCl₂
33.5% YEPD

Breaking Buffer
50 mM Na₃PO₄, pH 7.5
5% glycerol
1 mM EDTA
1 mM PMSF (Sigma)

YPD, 1 liter
10 g Yeast extract
20 g peptone
10 g dextrose

SDR, 1 liter
6.7 g YNB
400 μg biotin
182 g Sorbitol
10 g dextrose
10 g agar
50 mg each of glutamine, methionine,
lysine, leucine and isoleucine
2 g histidine assay mix

SDHR, 1 liter
SDR + 20 mg histidine

Example I

DNA Miniprep

| Solution I | | |
|---|---|---|
| 0.5 | ml | 20% sterile glucose (~50mM final concentration) |
| 1 | ml | 10mM EDTA, pH 8.0 |
| 0.25 | ml | 1M Tris•Cl, pH 8.0 |
| 8.25 | ml | dH$_2$O |
| 10 | ml | Total Volume (Add 10 mg lysozyme (Sigma) to 2.5ml solution I) |

Solution II Prepare Weekly
0.2 N NaOH
1% SDS
Solution III
3 M NaOAc, pH 4.8

1.5 mls of an overnight culture of E. coli DG75 cells were centrifuged 4°C for 30 seconds using a Beckman microfuge. The resulting supernatant was discarded, the pellet resuspended in 100 μl of solution I containing 4 mg/ml lysozyme, and the sample was incubated on ice for 30 minutes. 200 μl of solution II was added and gently vortexed to break apart protein clumps. The sample was again incubated on ice for 30 minutes. 150 μl of solution III was added to the sample. It was mixed and incubated on ice for 60 minutes. The sample was centrifuged as above for 5 minutes, and the supernatant was decanted to fresh tubes. 1 ml of absolute ethanol was added to the supernatant, and incubated at -20°C overnight. After incubation, the sample was centrifuged for 15 minutes as above, the supernatant discarded, and the pellet washed two times with 1 ml cold 70% ethanol. The pellet was then dried under vacuum and resuspended in 50μl 10mM Tris•Cl, 0.1mM EDTA. Samples were then frozen at -20°C for storage.

Example II

Creation of pHIL 301

Plasmid pA0804 is available in an E. coli host deposited at the Northern Regional Research Center, U. S. Department of Agriculture, Peoria, Illinois, accession number NRRL B-18114, which can be obtained by harvesting plasmid DNA, digesting with EcoRI, and recovering the 7.1 Kb fragment from preparative agarose gels. Plasmid pHIL 301 was created from pA0804 as follows: 0.21μg pA0804 was digested with 10 units of restriction endonuclease NaeI in 20 μl (total volume) of medium salts buffer (50mM NaCL, 10mM Tris•Cl pH 7.5, 10mM MgCl$_2$, 1mM dithiothreitol) containing bovine serum albumin at a final concentration of 100 μg/ml. Digestion was carried out for 70 minutes at 37°C. The sample was then heated to 70°C for 5 minutes and 30μl of dH$_2$O was added. A phenol:chloroform extraction was performed according to Maniatis et al, and the DNA precipitated by the addition of $\frac{1}{2}$ volume (25μl) of 7.5 M ammonium acetate, followed by the addition of 150μl of absolute ethanol and incubation at -70°C for 45 minutes. The DNA was collected by centrifugation at high speed for 15 minutes using a Hermle table top centrifuge. The DNA pellet was washed twice by the addition of 1ml of 70% ethanol and followed by centrifugation as described above. The pellet was then dried under vacuum and resuspended in 10μl of dH$_2$O. This solution contained what was designated "cleaved vector".

In a separate digestion Kan [R] Genblock (Pharmacia), containing the kanamycin resistance gene, Km[R], was digested with 10 units of the restriction enzyme HincII in 20μl (total volume) of medium salts buffer containing bovine serum albumin at a final concentration of 100 μg/ml. Digestion was carried out for 70 minutes at 37°C, and then the sample was heated for 5 minutes at 70°C. 30μl of dH$_2$O was added and a phenol:chloroform extraction performed as above. The DNA was then precipitated by the method described above and dried under vacuum. The sample was left in the dry state and designated "cleaved insert".

The dried, cleaved insert was resuspended in a total volume of 10μl containing the following: 5μl (about 0.1μg) of cleaved vector (described above), 2μl of 5 X ligase buffer (Bethesda Research Labs), 1 unit of T4 ligase (BRL), and bovine serum albumin at a final concentration of 100 μg/ml. Two control reactions were performed and contain the following: 1) the above ligation mix without cleaved insert DNA and 2) the above ligation mix without cleaved insert DNA and without ligase. The reactions were incubated at room temperature for about 65 hours.

Each of the samples was then used to transform E.coli strain DG75' as described in Dagart et al., Gene 6:23-28 (1979). Transformed cells were plated on LB medium containing ampicillin (100μg/ml) and kanamycin sulfate (50μg/ml), and incubated at 37°C overnight. Colonies present were derived from cells transformed with the ligation mixture of cleaved vector and cleaved insert combined. The plasmid content of the transformants was analyzed by the miniprep procedure as described in Example I, followed by PstI digestion and agarose gel electrophoresis. Two strains designated DG75' (pHIL 301) and DG75' (pHIL 302) were selected as having the Km[R] gene inserted into pA0804.

## Example III

### Construction of E. coli Plasmid Intermediates

The following linker was designed and synthesized by a DNA synthesizer from Applied Biosystems (Model 380A) using the β-cyanoethyl phosphoramidite method:

$$5'\text{-CATGAATTCAAAA-}3'$$
$$3'\text{-TTAAGTTTTTGTAC-}5'$$

This will insert into NcoI cut DNA, destroying the NcoI site and leaving the DNA with an EcoRI site. The two strands of the linker were phosphorylated, annealed, and ligated into NcoI cut, dephosphorylated pUC9:pL:IL2 DNA. Following ligation the DNA was cut with NcoI to inactivate any molecules without the linker. The sample was then used to transform DG75' as described in Example II, with selection for ampicillin resistance. 21 transformants were used to make minipreps of plasmid DNA as described in Example I, which were then cut with EcoRI and subjected to agarose gel electrophoresis. 14 of these contained and described new EcoRI site 5' to the beginning of the hIL-2 structural gene. Due to the preexisting EcoRI site at the 3' end of the hIL-2 gene, this new construct would allow removal of the hIL-2 gene on one EcoRI fragment.

## Example IV

### Construction of IL-2 gene in P. pastoris expression vector

Samples with the confirmed new EcoRI site from Example III were pooled, cut with EcoRI, and ligated into pHIL 301 from Example II that had been cut with EcoRI and dephosphorylated. DG75 was transformed with this DNA as described in Example II, with selection for kanamycin resistance (Km[R]). 24 Km[R] transformants were used to make minipreps of plasmid DNA as described in Example I, which were then cut with HindIII and BamH1 and subjected to agarose gel electrophoresis. Three of the isolates contained the hIL-2 gene in the proper orientation in pHIL 301. One of these was designated pHIL 333.

Example V

Transformation of Pichia pastoria

A. Vector preparation

10 μg of plasmid pHIL 333 obtained from E. coli DG75' was subjected to a complete digestion with BglII. This DNA fragment was used to transform Pichia pastoris strain GS115 (HIS4) deposited with the Northern Regional Research Center of the U.S. Department of Agriculture, accession number NRRL Y-15851. Transformation with vectors containing the histidinol dehydrogenase gene will complement this defect in the histidine pathway, changing the GS115 transformed cells to His[+]. Screening for transformants may therefore be easily accomplished by culturing the cells after transformation in a growth environment lacking histidine and recovering the cells capable of growing under this condition.

B. Cell Growth

Pichia pastoris GS115 (NRRL Y-15851) was inoculated into about 100 ml of YPD medium and shake cultured at 30°C for 12-20 hours. 100 ml of YPD medium was inoculated with seed culture to give an $OD_{600}$ of about 0.001. The medium was cultured in a shake flask at 30°C for about 12-20 hours. The culture was harvested when the $OD_{600}$ was about 0.2-0.3 (after approximately 16-20 hours) by centrifugation at 1500 g for 5 minutes using a Sorvall RC5C.

C. Preparation of Spheroplasts

The cells were washed once in 10ml of sterile water, and then centrifuged at 1500 g for 5 minutes. (Centrifugation is performed after each cell wash at 1500 g for 5 minutes using a Sorvall RT6000B unless otherwise indicated). The cells were then washed once in 10ml of freshly prepared SED, once in 10ml of sterile 1M sorbitol, and finally resuspended in 10ml of SCE buffer. 7.5μl of 3mg/ml Zymolyase (100,000 units/g obtained from Miles Laboratories) was added to the cell solution. The cells were then incubated at 30°C for about 6 minutes. (A reduction of 80% in $OD_{600}$ can be utilized as a correct time and concentration marker). The spheroplasts were washed once in 10 ml of sterile 1M sorbitol by centrifugation at 1,000 g for 5-10 minutes. (The time and speed for centrifugation may vary; centrifuge enough to pellet the spheroplasts but not so much they rupture from the force). 10ml of sterile CaS was used as a final cell wash, and the cells were centrifuged again at 1,000 g for 5-10 minutes and resuspended in 0.6ml of CaS.

D. Transformation

GS115 cells were transformed with 10μg of the linear pHIL 333 vector using the spheroplast transformation technique of Sreekrishna et al. in Gene 59, 115-125 (1987). DNA samples were added (up to 20μl volume) to 12 x 75 mm sterile polypropylene tubes. (DNA should be in a suitable buffer, such as TE buffer); 100 μl of spheroplasts were added to each DNA sample and incubated at room temperature for about 10 minutes. 1 ml of PEG solution was added to each sample and incubated at room temperature for about 10 minutes and centrifuged at 1,000 g for 5-10 minutes. SOS (150 μl) was added to the pellet, and incubated for 20 minutes at room temperature. Finally, 850 μl of 1M sorbitol was added.

E. Regeneration of Spheroplasts

A bottom agar layer of 20 ml of regeneration agar SDR was poured per plate at least 30 minutes before transformation samples were ready. In addition, 8 ml aliquots of regeneration agar were distributed to 15 ml conical bottom corning tubes in a 45°C bath during the period that transformation samples were in SOS.

Aliquots of 50, 250 or 800 μl of the transformed sample was added to the 8 ml aliquots of melted regeneration agar held at 45° C and poured onto plates containing the solid 20 ml bottom agar layer. The plates were incubated at 30° C for 3-5 days.

F. Selection of Transformants

Transformants were selected for by culturing on SDR, a media lacking histidine. Cultures which grew in the absence of histidine were additionally screened for the "methanol slow" phenotype (indicating site-selective integration). The transformed GS115 cells showing evidence of both phenotypes were then cultured and assayed for the production of hIL-2. Pichia pastoris GS115/pHIL 333-14 was selected by this process.

Example VI

Detection and Confirmation of the Presence of IL-2

Electroimmunoblots - Proteins separated in polyacrylamide gels, including standard IL-2 (Collaborative Research), were transferred electrophoretically to nitrocellulose (BA85, Schleicher and Schuell) as described in Towbin et al., P.N.A.S. 76, 4350 (1979) at 300 mA for 2-4 hr. Immunoblots were stained for protein using Ponceau S (Sigma) according to directions and photographed. The nitrocellulose was rinsed in TBST (0.15 M NaCl in 10 mM Tris•Cl, pH 7.4 containing 0.05% Tween 20). Additional protein-binding sites on the nitrocellulose were blocked by incubation in blocking buffer (5% nonfat dry milk, 0.1 mg/ml thimerosal, Johnson et al., Gene Anal. Techn. 1, 3 (1984) in TBST with 0.05% Tween 20 (Batteiger et al., J. Immunol. Methods 55, 297 (1982)) for 1-18 hrs. at 37° C. The nitrocellulose was then incubated in mouse monoclonal antibody to IL-2 (Genzyme) at 10 μg/ml blocking buffer for 1.5 hr. at 22° C with gentle rocking. After washing 6 times for 5 minutes in TBST, the nitrocellulose was incubated in goat antimouse IgG (Kirkegaard and Perry Labs) at 1 μg/ml blocking buffer for 45 minutes at 22° C with gentle rocking. After washing in TBST as above, the nitrocellulose was incubated in alkaline phosphatase-conjugated rabbit antigoat IgG (Kirkegaard and Perry Labs) at 0.25 μg/ml blocking buffer for 45 minutes at 22° C with gentle rocking. After washing in TBST as above, the nitrocellulose was immersed in the BCIP/NBT alkaline phosphatase substrate (Kirkegaard and Perry Labs) and rocked gently for 10-30 minutes until color developed. An intense blue-black reaction product develops where hIL-2 is present in the gel. Under these conditions ≥ 5ng hIL-2 can be detected.

Enzyme-Linked Immunosorbent Assay (ELISA) - The assay used was a sandwich ELISA in which microtiter wells of 96-well ELISA plates (Corning) were coated for 2 h at 37° C with mouse monoclonal antibody to hIL-2 (Genzyme) at 10 μg/ml, 0.05 M $Na_2CO_3$ - $NaHCO_3$, pH 9.5. After rinsing with TBST, additional protein binding sites were blocked using the blocking buffer described above. The plates were washed 5 times in TBST, then 50 μl/well of control hIL-2 (Collaborative Research) or of test solutions was added, and the plates incubated at 22° C on a shaker (Dynatech) for 1.5h. The plates were washed as above, and 50 μl/well of rabbit antibody to hIL-2 (Collaborative Research) at 10 μg/ml of blocking solution were added. After incubation for 45 min. on the shaker, the plates were washed 5 times as above, 50 μl/well of biotin-labeled goat antirabbit IgG (Kirkegaard and Perry Labs) at 0.5 μg/ml blocking solution was added, and the plates were incubated on the shaker for 45 min. After washing 5 times as above, 50 μl/well of horseradish peroxidase-conjugated streptavidin (Kirkegaard and Perry Labs) at 0.5 μg/ml blocking buffer was added, the plates were incubated for 45 min. on the shaker, and again washed 5 times as above in TBST. Finally 100 μl/well of ABTS Peroxidase Substrate Solution (Kirkegaard and Perry Labs) was added, plates were incubated on the shaker for 20 min., then the reaction was stopped by adding 100 μl/well of 2% oxalic acid. A green reaction product was formed in the wells, and the $A_{405}$ was measured using the Kinetic Microplate reader (Molecular Devices). Under these conditions, ≥ 2 ng/ml IL-2 can be detected. See data on IL-2 expression contained in Table 2. (Assuming protein concentrations in the cell extracts of 1 mg/ml, and 10% solubility of the IL-2, the measured amount of IL-2 corresponded to 0.04-0.08% of the total soluble cellular protein, or 4-8 μg IL-2/ml (4-8 mg/l) in shake flasks).

Table 2

| Estimation by ELISA of IL-2 Concentration in Pichia Cell Extracts | | |
|---|---|---|
| Sample | IL-2 Gene (+ or -) | Concentration of IL-2 (ng/ml) |
| 11 | + | 440 |
| 12 | + | 447 |
| 13 | + | 437 |
| 14 | + | 560 |
| 15 | + | 780 |
| 16 | + | 762 |
| 17 | + | 630 |
| 18 | + | 633 |
| 19 | + | 808 |
| 20 | + | 515 |
| 21 | - | <3.5 |
| 22 | - | <3.5 |
| 23 | - | <3.5 |

The amount of IL-2 present in cells at different times during methanol induction (0.5% v/v) was also estimated by ELISA (Table 3).

Table 3

| Estimation by ELISA of IL-2 in Cell Extracts of Pichia During the Course of Methanol Induction | | |
|---|---|---|
| Cells | Hours in Methanol | Concentration of IL-2 (ng/ml) |
| Control (IL-2 -) | 12 | <2.5 |
| | 48 | <2.5 |
| IL-2 + | 12 | 280 |
| | 48 | 120 |

Biological Activity - The biological activity of soluble, intracytoplasmic extracts from cells transformed with control plasmids or which hIL-2 gene-containing plasmids was assessed during the course of methanol induction of hIL-2 expression (Table 4). The level of biological activity in relation to the amount of immunoreactive IL-2 protein detected corresponds to 4-20 X$10^6$ BRMP units/mg protein. This value compares well with a value of 5.8 x $10^6$ units/mg protein of the IL-2 standard (Collaborative Research lot 88-1105) used in these experiments.

The IL-2 microassay was performed according to Gillis et al., J. Immun. 120, 2027 (1978). Briefly, CTLL-2 cells (ATCC No. TIB 214), that are IL-2-dependent and routinely cultured in RPMI 1640 (Gibco) with 10% heat-inactivated fetal bovine serum (Gibco), 10 mM Hepes (Gibco), 2 mM Glutamine (Gibco) and 1% antibiotic-antimycotic solution (Sigma) with 5% rat T-cell polyclone IL-2 (Collaborative Research) were collected by centrifugation at 300 x g in a Sorvall RT6000 centrifuge, washed 3 times in culture medium without IL-2, and resuspended in medium without hIL-2 at 2 x $10^5$ cells/ml. Aliquots of 100 $\mu$l/well were distributed into 96-well flat-bottomed Cell Wells (Corning). 100 $\mu$l of medium containing IL-2 (Collaborative Research) at 0.2 - 500 units/ml or test solutions of cell extract concentrations ranging between 0.005 and 20% by volume in the well were added and the plates were incubated in a humidified $CO_2$ incubator (Queue) at 37° C for 24h. During the last 4 hr, 1 $\mu$Ci/well of $^3$H-thymidine (Amersham) was added. The cells were harvested onto glass fiber filters (Skatron) and the amount of thymidine incorporated into high molecular weight material was quantitated by counting the filter disks in a Beckman LS 5801 scintillation counter. Under these conditions, background incorporation without added IL-2 is <1000 cpm, and maximal

EP 0 339 568 A1

incorporation with authentic IL-2 ranged between 60,000 and 100,000 CPM.

Table 4

| Biological Activity of IL-2 in Cell Extracts of Pichia | | | |
|---|---|---|---|
| Cell Extract | Hours in Methanol | Concentration of Extract (%v/v) | Incorporated $^3$H-Thymidine (cpm) |
| None | - | - | 388 |
| Control (IL-2 -) | 12 | 0.1 | 1220 |
| | | 2.0 | 941 |
| | 48 | 0.1 | 255 |
| | | 2.0 | 459 |
| IL-2 + | 12 | 0.1 | 20190 |
| | | 2.0 | 52176 |
| | 48 | 0.1 | 6882 |
| | | 2.0 | 87780 |
| Authentic IL-2 | - | (10 BRMP units/ml) | 160024 |

The examples have been provided merely to illustrate the practice of the invention and should not be read so as to limit the scope of the invention or the appended claims in any way. Reasonable variations and modifications, not departing from the essence nd spirit of the invention, are contemplated to be within the scope of patent protection desired and sought.

**Claims**

1. A process for the enhanced production of hIL-2 comprising

a) transforming a methylotrophic yeast with at least one vector having at least one expression cassette containing a structural gene for hIL-2, operably linked to a regulatory region and a 3′ termination sequence; and thereafter

b) culturing the resulting transformed yeast strain under suitable conditions to obtain the production of said hIL-2 protein.

2. The process of claim 1 wehrein said vector is selected from plasmids or linear integrative site-specific vectors.

3. The process of claim 2 wherein said linear integrative site-specific vector contains the following serial arrangement:

a) a first insertable DNA fragment,

b) at least one marker gene, and at least one expression cassette containing a structural gene for hIL-2, operably linked to a regulatory region and a 3′ termination sequence, and

c) a second insertable DNA fragment;

wherein the order of the marker gene and cassette of component (b) may be interchanged.

4. The process of claim 3 wherein the first insertable DNA fragment and the second insertable DNA fragment are derived from the DNA sequence of a gene isolated from Pichia pastoris and selected from AOX1, p40, DHAS and HIS4.

5. The process claim 4 wherein said expression cassette comprises

a) a regulatory region selected from AOX1, p40, DHAS and HIS4, isolated from Pichia pastoris, acid phosphatase, galactosidase, alcohol dehydrogenase, cytochrome c, alpha-mating factor and glyceraldehyde 3-phosphate dehydrogenase isolated from Saccharomyces cerevisiae operably linked to

b) a structural gene for hIL-2 operably linked to

c) a 3′ termination sequence from Pichia pastoris selected from the 3′ termination sequences isolated from the AOX1 gene, p40 gene, DHAS gene and HIS4 gene.

14

6. The process of claim 3 wherein said marker gene is selected from HIS4 and ARG4, isolated from Pichia pastoris, SUC2 isolated from Saccharomyces cerevisiae, G418$^R$/kanamycin resistant gene from bacterial transposon Tn601 or Tn903 gene.

7. The process of claim 3 wherein said vector comprises

a) a first insertable DNA fragment which is about one kilobase of the 5′ AOX1 regulatory region isolated from Pichia pastoris operably linked to

b) a structural gene for hIL-2, operably linked to

c) the 3′ termination sequence of AOX1 isolated from Pichia pastoris ligated to

d) a marker gene which is HIS4 isolated from Pichia pastoris ligated to

e) a marker gene which is kanamycin resistant from transposon Tn903 or Tn601,

f) a second insertable DNA fragment which is about 0.65 kilobases of the 3′ AOX1 termination sequence.

8. A linear integrative site-specific vector comprising the following serial arrangement
a) a first insertable DNA fragment,
b) at least one marker gene and at least one expression cassette containing a structural gene for hIL-2, operably linked to a regulatory region and a 3′ termination sequence, and
c) a second insertable DNA fragment;
wherein the order of the marker gene and cassette of component (b) may be interchanged.

9. The vector of claim 8, wehrein said vector is as defined in any of claims 4 to 7.

10. Methylotrophic yeast transformed with at least one vector containing at least one expression cassette comprising a regulatory region operably linked to a structural gene for hIL-2, operably linked to a 3′ termination sequence.

11. The methylotrophic yeast of claim 10 wherein the yeast is Pichia pastoris.

12. The methyltrophic yeast of claim 11 wherein the yeast is Pichia pastoris strain GS115.

13. The yeast of any of claims 10 - 12 wherein said yeast is transformed with at least one linear integrative site-specific vector as defined in any of claims 3 - 9.

14. Picha pastoris, GS115/pHIL333.

15. Pichia pastoris GS115 transformed as in claim 13 wherein said GS115 is transformed with more than one copy of said linear integrative site-specific vector.

FIG. 1

*FIG. 2*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 152 358 (TRANSGENE S.A.) * Abstract * -- | 1 | C 12 N 15/00 C 12 P 21/00 C 12 N 1/16 //(C 12 N 1/16 C 12 R 1:84) |
| A | EP - A1 - 0 142 268 (JAPANESE FOUNDATION FOR CANCER RESEARCH) * Abstract * -- | 1 | |
| D,A | BIOTECHNOLOGY, vol. 5, no. 5, May 1987 (New York) J.M.CREGG et al. "High-Level Expression and Efficient Assembly of Hepatitis B Surface Antigen in the Methylotrophic Yeast, Pichia pastoris" pages 479-485 * Totality * -- | 1,10-15 | |
| A | EP - A2 - 0 183 070 (PHILLIPS PETROLEUM COMPANY) * Abstract; claims 1,3 * -- | 1,10-14 | TECHNICAL FIELDS SEARCHED (Int. Cl.4) |
| D,A | EP - A1 - 0 226 752 (PHILLIPS PETROLEUM COMPANY) * Abstract * -- | 1-3,8 | C 12 N C 12 P |
| D,A | EP - A2 - 0 183 071 (PHILLIPS PETROLEUM COMPANY) * Abstract * ---- | 1 | |

The present search report has been drawn up for all claims

| Place of search VIENNA | Date of completion of the search 02-08-1989 | Examiner WOLF |
|---|---|---|